(19) **Europäisches Patentamt**

**European Patent Office**

**Office européen des brevets**

(11) **EP 1 023 939 B1**

(12) **EUROPÄISCHE PATENTSCHRIFT**

(45) Veröffentlichungstag und Bekanntmachung des Hinweises auf die Patenterteilung:
**17.04.2002 Patentblatt 2002/16**

(51) Int Cl.7: **B01J 8/24**, C07C 17/156, C07C 19/045, B01J 8/00

(21) Anmeldenummer: **00100199.9**

(22) Anmeldetag: **13.01.2000**

(54) **Verfahren für die Herstellung von 1,2-Dichlorethan aus der Oxichlorierung**

Process for producing 1,2-Dichloroethane by oxychlorination

Procédé pour produire du 1,2-Dichloroethane par oxychloration

(84) Benannte Vertragsstaaten:
**BE DE FR IT NL**

(30) Priorität: **28.01.1999 DE 19903335**

(43) Veröffentlichungstag der Anmeldung:
**02.08.2000 Patentblatt 2000/31**

(73) Patentinhaber: **Vinnolit Technologie GmbH & Co. KG**
**84504 Burghausen (DE)**

(72) Erfinder:
• **Ertl, Horst, Dr.**
**84524 Neuötting (DE)**
• **Kammerhofer, Peter**
**84508 Burgkirchen (DE)**
• **Schwarzmaier, Peter**
**84556 Kastl (DE)**
• **Mielke, Ingolf, Dr.**
**84508 Burgkirchen (DE)**

(74) Vertreter:
**Forstmeyer, Dietmar, Dr. rer. nat., Dipl.-Chem. et al**
**Boeters & Bauer,**
**Bereiteranger 15**
**81541 München (DE)**

(56) Entgegenhaltungen:
DE-A- 4 305 001          DE-A- 19 753 165
US-A- 3 679 373          US-A- 3 816 554

EP 1 023 939 B1

## Beschreibung

Die Erfindung betrifft ein Verfahren für die Herstellung von 1,2-Dichlorethan aus der Oxichlorierung und eine Vorrichtung zu dessen Durchführung.

Unter Oxichlorierung versteht man allgemein die Umsetzung eines Alkens mit Chlorwasserstoff und Sauerstoff oder einem Sauerstoff enthaltenden Gas wie Luft, unter Bildung eines gesättigten chlorierten Alkans. Die Reaktion erfolgt nach der folgenden Gleichung, die beispielsweise die Umsetzung von Ethen zu 1,2-Dichlorethan, im folgenden "EDC" genannt, zeigt

$$C_2H_4 + 2HCl + \tfrac{1}{2}\, O_2 \Longleftarrow\Longrightarrow Cl\text{-}CH_2\text{-}CH_2\text{-}Cl + H_2O.$$

In einer häufig im großindustriellen Ausmaß ausgeübten Ausführungsform dieses Verfahrens dient als Katalysator ein Wirbelbett, wobei der Katalysator im wesentlichen aus Kupferchlorid auf einem Aluminiumoxidträger besteht. In dieses Katalysatorbett werden - getrennt voneinander - das Ethen und der Sauerstoff beziehungsweise das sauerstoffhaltige Gas eingeleitet, wobei der Chlorwasserstoff üblicherweise zusammen mit dem Sauerstoff beziehungsweise dem sauerstoffhaltigen Gas eindosiert wird. Man sorgt so dafür, daß die Reaktanden zunächst erst mit dem Wirbelbett in Kontakt treten, bevor sie zur Reaktion aufeinandertreffen. Man vermeidet so die Bildung explosionsfähiger Gemische.

Aus DE-C-195 05 664 (AU-A 9528810) ist eine Vorrichtung und ihre Verwendung zur Oxichlorierung bekannt, die gekennzeichnet ist durch einen Reaktor mit einem abgegrenzten Katalysatorfließbett, in dem innerhalb dieses Katalysatorfließbetts eine erste Gaseinleitung (Verteilerrohre)angebracht ist, die über den gesamten Querschnitt des Reaktors verteilt Düsen enthält. Die Düsen münden dabei in Rohre, die den austretenden Gasstrom im wesentlichen im Gegenstrom zu dem Gasstrom führen, der den Katalysator fluidisiert. Der Raum zwischen den unteren Enden dieser Rohre und den oberen Enden der Rohre, die in der Begrenzung des Katalysatorfließbetts zum restlichen Reaktor mit der zweiten Gaseinleitung eingearbeitet sind, bildet dabei eine Mischzone, die so groß bemesssen ist, daß einerseits die Vermischung der Reaktanden mit dem Katalysator hier bereits stattfinden kann und daß andererseits eine hohe gegenseitige erosive Beanspruchung der Rohre sowie der unteren Begrenzung nicht mehr gegeben ist.

Bevorzugte Ausgestaltungen dieser Vorrichtung besitzen dabei Rohre, die durch die Begrenzung zum Katalysatorfließbett hindurchführen, in denen Düsen unterhalb der Begrenzung, aber oberhalb dem unteren Ende der Rohre angeordnet sind. Weiterhin zeichnen sich diese Düsen dadurch aus, daß sie in einem so großen Abstand zum oberen Ende der Rohre angebracht sind, daß sich die nach oben gerichtete Strömungsgeschwindigkeit der Gasstrahlen aus den Düsen bis zum oberen Ende der Rohre über den jeweiligen Querschnitt eines Rohres vergleichmäßigt hat. Die Düsen sind dabei etwa in einer Entfernung von einem Durchmesser eines Rohres am unteren Ende der Rohre angebracht und die Länge der Rohre ist so groß gewählt, daß sich die nach unten gerichtete Strömungsgeschwindigkeit der Gasstrahlen aus den Düsen bis zum unteren Ende der Rohre über den jeweiligen Querschnitt eines Rohres vergleichmäßigt hat. Die Düsen haben dabei unterschiedliche Durchmesser, damit die über die Gaseinleitung geführte Gasmenge gleichmäßig über den Querschnitt des Reaktors verteilt wird.

Verfahren mit ähnlichen Vorrichtungen sind aus DE-U-91 16 161 und WO-A-94/19099 (ZA-A 9401086) bekannt.

In dem Dokument US-A-3,679,373 wird eine Vorrichtung mit Wirbelbetten beschrieben, die eine Reaktionskammer und Einlässe für Reaktanden umfaßt, die auf zwei verschiedenen Ebenen angeordnet sind. Zu chlorierendes Mittel, Chlorierungsmittel und Sauerstoff werden getrennt voneinander in die Vorrichtung eingeführt.

GB-A-1033639 betrifft ein Verfahren zur Oxichlorierung eines Olefins, das die Umsetzung eines gasförmigen Gemisches von einem Olefin, Chlorwasserstoff und Sauerstoff oder einem sauerstoffhaltigen Gas in Gegenwart eines Wirbelbettes zur Herstellung eines oxichlorierten Olefins umfaßt.

Alle bekannten Verfahren und die damit verbundenen Vorrichtungen sind aufwendig konstruiert und erlauben nur ein sehr unkontrolliertes Einleiten der Eduktgasströme in den Reaktor.

Es bestand daher die Aufgabe, ein Verfahren zur Verfügung zu stellen, bei dem die Zuführung der Edukte in den Reaktor möglichst einfach und gleichzeitig fein verteilt durchgeführt werden kann.

Gegenstand der Erfindung ist ein Verfahren zur Herstellung von 1,2-Dichlorethan durch Umsetzung von Ethen mit Chlorwasserstoff und Sauerstoff oder einem sauerstoffhaltigen Gas an einem kupferhaltigen Katalysator im Wirbelbett, dadurch gekennzeichnet, daß die Einleitung mindestens einer der Eduktströme Chlorwasserstoff und Sauerstoff, direkt in das Wirbelbett, über Zuleitungen aus porösem gasdurchlässigen Formkörper erfolgt und die Einleitung des Ethens und des Kreisgasstroms über einen Boden, der aus porösem gasdurchlässigem Material gefertigt, oder mit Formkörper aus porösem gasdurchlässigem Material versehen ist, erfolgt.

Erfindungsgemäß erfolgt somit die Zuführung der Reaktionspartner Ethen einerseits und Sauerstoff andererseits, wobei hierunter auch ein sauerstoffhaltiges Gas verstanden werden soll, in der Form, daß mindestens einer dieser Reaktionspartner über einen porösen Formkörper in feinverteilter Form dem Wirbelbett zugeführt wird. Der Chlorwasserstoff kann in an sich bekannter Weise einem oder beiden der genannten Reaktionsteilnehmer zudosiert beziehungsweise zugemischt werden.

Vorzugsweise werden sowohl der Sauerstoff einerseits als auch das Ethen andererseits in derart feinverteilter Form in das Katalysatorwirbelbett eingespeist, wobei jeweils, wie vorstehend ausgeführt, der Chlorwasserstoff mit einem oder beiden der Reaktionspartner dem Wirbelbett zugeführt werden kann.

Vorzugsweise sind die Zuleitungen in Form von Rohren ausgestaltet, ähnlich einer Filterkerze oder einer Reihe von - beispielsweise parallel angeordneten - rohrförmigen Elementen aus porösen Materialien. Geeignet sind beispielsweise Fritten aus Glas oder Keramik, wie sie auch aus der Labortechnik bekannt sind. Vorzugsweise eignen sich dünne poröse Elemente aus gesinterten Metallen, die die entsprechende notwendige chemische und thermische Resistenz aufweisen. Hierzu eignen sich die im chemischen Apparatebau eingeführten Materialien wie VA-Stahllegierungen oder hochkorrosionsfeste Legierungen, wie sie unter den Bezeichnungen ® INCONEL (Marke der Firma Inco Ltd.; Nickel-Chrom-Legierung), ® MONEL (Marke der Firma Inco Ltd.; Nickel-Kupfer-Legierung), ® HASTELLOY (Nickel-Legierung) handelsüblich sind. Sofern ein Gegenstrom der Reaktionsteilnehmer beabsichtigt ist, wird die Gaszuführung mit dem porösen Formkörper innerhalb des Wirbelbettes so ausgestaltet, daß der Gasaustritt im Gegenstrom zu dem Gasstrom durch die untere Begrenzung des Wirbelbettes erfolgt. Sofern dieser Gegenstrom nicht gewünscht wird, kann die Gaszuführung in andere Strömungsrichtungen gerichtet sein oder in alle Richtungen erfolgen.

Die Poren der porösen Filterelemente der Begasungsvorrichtung besitzen dabei zweckmäßigerweise einen Durchmesser von 0,5 bis 50 μm, vorzugsweise 5 bis 20 μm.

Ein weiterer Aspekt der Erfindung betrifft die untere Begrenzung des Wirbelbettes, die erfindungsgemäß als poröser Formkörper ausgebildet ist. Dasu eignen sich beispielsweise Fritten aus Glas oder Keramik, wie sie auch aus der Labortechnik bekannt sind. Vorzugsweise eignen sich dünne poröse Elemente aus gesinterten Metallen, die die entsprechende notwendige chemische und thermische Resistenz aufweisen. Hierzu eignen sich die im chemischen Apparatebau eingeführten Materialien wie VA-Stahllegierungen oder hochkorrosionsfeste Legierungen, wie sie unter den Bezeichnungen ® INCONEL (Marke der Firma Inco Ltd.; Nickel-Chrom-Legierung), ® MONEL (Marke der Firma Inco Ltd.; Nickel-Kupfer-Legierung), ® HASTELLOY (Nickel-Legierung) handelsüblich sind.

Ein weiterer Gegenstand der Erfindung ist eine Vorrichtung zur Durchführung des Verfahrens umfassend einen mit einem Wirbelbett ausgerüsteten Reaktor (2), welcher für die Einleitung der Eduktströme mit zwei Leitungen (1, 3) und für die Ableitung der Produkte mit mindestens einer Leitung (5) ausgestattet ist, dadurch gekennzeichnet, daß die Leitungen zur Zuführung der Edukte aus porösem Material gefertigt sind.

Bevorzugt ist eine Ausführungsform der Vorrichtung, dadurch gekennzeichnet, daß vor der Ableitung der Produkte über eine Leitung (5) diese mit einer Filtervorrichtung (4) aus porösem Material gefiltert werden.

Bevorzugt ist weiterhin eine Ausführungsform der Vorrichtung, dadurch gekennzeichnet, daß für die Einleitung des Ethylens und des Kreisgasstroms der Reaktor (2) mit einem Boden (12), der aus porösem gasdurchlässigem Material gefertigt, oder mit Formkörper aus porösem gasdurchlässigem Material versehen ist, ausgerüstet ist.

Die Erfindung bringt eine Reihe von Vorteilen mit sich:

Die Elemente aus porösen Material bewirken, daß die zugeführten Gase in die Katalysatorwirbelschicht als sehr feine Blasen zugeführt werden. Diese können naturgemäß sehr viel leichter mit der Katalysatoroberfläche in Kontakt treten und reagieren deshalb sehr viel rascher mit dem Reaktionspartner. Hierdurch wird die Reaktion entscheidend besser kontrollierbar und somit selektiver, das heißt, die Bildung von Nebenprodukten wird zurückgedrängt und die Ausbeute erhöht. Durch die Einleitung von feinen Gasblasen wird außerdem die Gefahr der Unkontrollierbarkeit der Reaktion wesentlich vermindert, was eine weitere Erhöhung der Sicherheit der Reaktion bedeutet. In gleicher Weise wirkt auch die dadurch erhaltene homogene Gasverteilung über den gesamten Reaktorquerschnitt, der ebenfalls den Stoffübergang verbessert. Die sehr feinen Gasblasen erlauben es weiterhin, den Abstand zwischen den Kühlelementen in der Wirbelschicht wesentlich zu verringern, so daß erheblich mehr an Kühlfläche pro Volumeneinheit in der Reaktionszone zur Verfügung steht. Diese erhöhte Kühlkapazität bewirkt ihrerseits eine besser kontrollierbare Reaktion, andererseits kann dadurch das Reaktorvolumen signifikant verringert werden.

Ein weiterer erheblicher Vorteil liegt darin, daß bei dem erfindungsgemäßen Verfahren durch die Gaszuführungsvorrichtungen aus porösem Material bei einer Unterbrechung der Zuführung an Reaktionsgasen kein feinteiliger Katalysator eindringen kann. Man spart so das bisher erforderliche Vorhalten eines Spülgases wie Stickstoff, dessen Volumen die Abgasverwertung unnötig belastet hat.

Eine besonders kompakte Bauweise des Oxichlorierungsreaktors wird dadurch ermöglicht, daß der Katalysator im Reaktor durch eine Feinfiltration weitgehend zurückgehalten wird, wobei vorzugsweise Grob- und Feinanteile des Katalysators in einem Schritt zurückgehalten werden. Diese Filtration mittels porösem gasdurchlässigen Formkörper kann beispielsweise in Form von Filterkerzen, Schlauch- oder Patronenfiltern erfolgen. Durch die Feinfiltration werden Teilchen > 1 μm zurückgehalten. Es können so die bisher erforderlichen Zyklone für die Zurückhaltung des Katalysators eingespart werden.

Durch die gleichmäßige Strömung der Reaktionsgase wird auch der Abrieb im Katalysator erheblich verringert. Hierdurch bleibt das Korngrößenspektrum im Katalysator über lange Zeiten im wesentlichen konstant, was wiederum zu einer Vergleichmäßigung der Reaktion beiträgt. Außer den bereits genannten Vorteilen hinsichtlich der verbesserten Selektivität und Sicherheit kommt hinzu, daß die Standzeiten der Anlage beträchtlich verlängert werden und teuere Ausfall- und Wartungszeiten verringert werden.

Im übrigen wird das Oxichlorierungsverfahren in an sich bekannter Weise durchgeführt:

Die Temperaturen in der Reaktionszone des Reaktors liegen zwischen 200 °C und 270 °C, vorzugsweise zwischen 215 °C und 230 °C, besonders bevorzugt zwischen 220 °C und 225 °C. Der dabei eingestellte Druck liegt bei $2,5 \times 10^5$ bis $5 \times 10^5$ Pa, vorzugsweise $3 \times 10^5$ bis $4 \times 10^5$ Pa, besonders bevorzugt bei $3,4 \times 10^5$ bis $3,5 \times 10^5$ Pa (jeweils Überdruck).

Dabei werden pro Mol Ethan 1,5 bis 2,5 mol, bevorzugt 1,8 bis 2,1 mol Chlorwasserstoff eingesetzt, sowie 0,2 bis 0,9 mol, bevorzugt 0,4 bis 0,7 mol Sauerstoff, wobei - in an sich bekannter Weise - darauf geachtet wird, daß das Ethen beziehungsweise der Sauerstoff erst mit dem Katalysator in Kontakt tritt, bevor es auf den anderen Reaktionspartner trifft, wie es beispielsweise in DE-C-195 05 664 gelehrt wird.

Alternativ dazu kann auch in anderen bekannten Weisen verfahren werden, so daß explosive Gasgemische vermieden werden.

Auch die Aufarbeitung des Reaktionsgases erfolgt in üblicher Weise. Hierzu kann beispielsweise auf die eingangs genannten Druckschriften verwiesen werden, deren diesbezügliche Offenlegung Teil der vorliegenden Anmeldung sein soll.

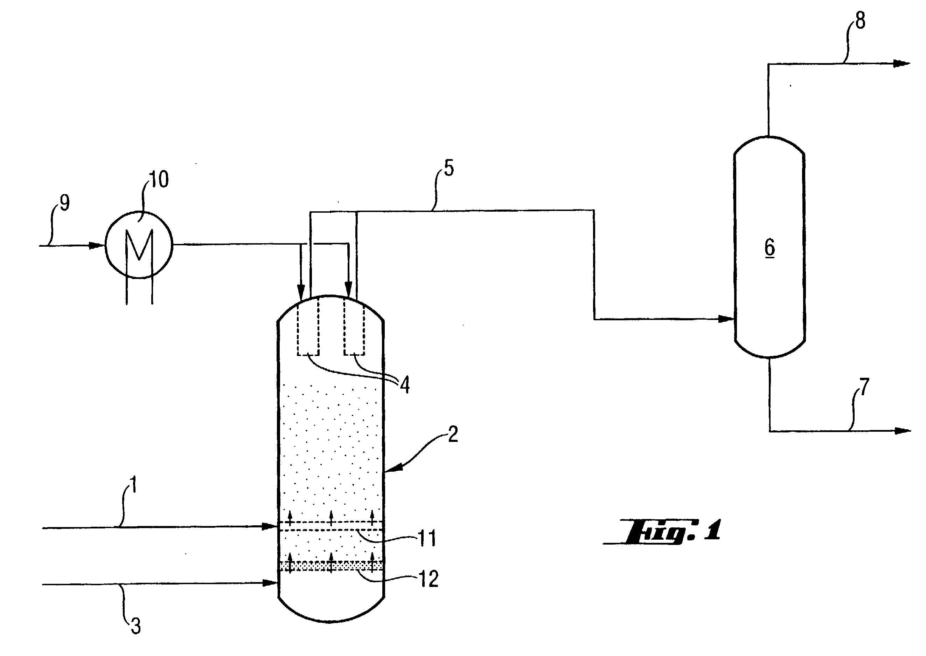

Im folgenden Beispiel soll die Erfindung noch näher erläutert werden. Die in Klammern angegebenen Zahlen verweisen dabei auf Figur 1.

**Beispiel 1:**

5910 Nm³/h Chlorwasserstoff mit einer Temperatur von 150 °C und 1600 Nm³/h Sauerstoff mit einer Temperatur von 110 °C wurden über die Leitung (1), über waagrecht angeordnete poröse rohrförmige Metallelemente (11), gemeinsam in den Reaktor (2), direkt in den unteren Teil des Wirbelbetts, eingeleitet. 3000 Nm³/h Ethylen wurden gemeinsam mit dem Kreisgas auf 150 °C erhitzt und über die Leitung (3) dem Reaktor (2) zugeführt. Die Einleitung dieses mit Ethen angereicherten Kreisgasstroms erfolgte über einen Boden, der mit Formkörper aus porösem Sintermetall ausgerüstet ist (12). Im Reaktor (2) befanden sich 40 t Wirbelbett-Katalysator (Aluminiumoxid mit einem Kupfergehalt von 4 Gew.-%) mit folgender Kornverteilung:

| Korngröße [µm] | Anteil (Durchgang) [Gew.-%] |
|---|---|
| < 20 | 4 |
| < 32 | 6 |
| < 41 | 26 |
| < 50 | 54 |
| < 61 | 82 |
| < 82 | 96 |

Die Reaktionswärme wurde über einen Heißwasserkreislauf unter Dampfgewinnung abgeführt. Das Reaktionsgas durchströmte nach Verlassen des Wirbelbetts zur Abscheidung von mitgerissenen Katalysatorteilchen im oberen Teil des Reaktors den Feinstfilter (4), in dem der Katalysator praktisch vollständig abgeschieden wurde. Das vom Katalysator befreite Reaktionsgas mit einer Temperatur von 220 °C wurde über die Leitung (5) in die Quenchkolonne (6) geleitet, wo das Produktionswasser kondensiert und über die Leitung (7) der Abwasseraufarbeitung zugeführt wurde. Der Kupfergehalt im Quenchwasser betrug < 0,05 mg/l. Der Kopfstrom, bestehend im wesentlichen aus EDC und Kreisgas, wurde über die Leitung (8) der EDC-Aufarbeitung zugeführt.

Der Feinstfilter (4) wurde differenzdruckgesteuert über die Leitung (9) mit Stickstoff, der im Vorheizer (10) auf 180 °C erhitzt wurde, abgereinigt. Die Rückhalterate betrug > 99,99 %. Nach dreimonatigem Betrieb konnte keine wesentliche Veränderung der Korngrößenverteilung im Wirbelbett festgestellt werden.

**Patentansprüche**

1. Verfahren zur Herstellung von 1,2-Dichlorethan durch Umsetzung von Ethen mit Chlorwasserstoff und Sauerstoff oder einem sauerstoffhaltigen Gas an einem kupferhaltigen Katalysator im Wirbelbett, **dadurch gekennzeichnet, daß** die Einleitung mindestens einer der Eduktströme Chlorwasserstoff und Sauerstoff, direkt in das Wirbelbett, über Zuleitungen aus porösem gasdurchlässigen Formkörper erfolgt und die Einleitung des Ethylens und des Kreisgasstroms über einen Boden, der aus porösem gasdurchlässigem Material gefertigt, oder mit Formkörper aus porösem gasdurchlässigem Material versehen ist, erfolgt.

2. Verfahren nach Anspruch 1, **dadurch gekennzeichnet, daß** die porösen Formkörper aus Glas oder Keramik bestehen.

3. Verfahren nach Anspruch 1, **dadurch gekennzeichnet, daß** die porösen Formkörper aus gesinterten Metallen bestehen.

4. Verfahren nach Anspruch 1 bis 3, **dadurch gekennzeichnet, daß** die porösen Formkörper Poren mit einem Durchmesser von 0,5 bis 50 µm besitzen.

5. Verfahren nach Anspruch 1 bis 4, **dadurch gekennzeichnet, daß** im Reaktor die Gaszuführung durch die porösen Formkörper in alle Richtungen erfolgen kann.

6. Verfahren nach Anspruch 1 bis 5, **dadurch gekennzeichnet, daß** der aus dem Wirbelbett mitgerissene Katalysator durch Feinfilter aus porösem gasdurchlässigen Formkörper im Reaktor weitgehend zurückgehalten wird.

7. Verfahren nach Anspruch 1 bis 6, **dadurch gekennzeichnet, daß** durch die Feinfiltration Teilchen > 1 µm zurückgehalten werden.

8. Verfahren nach Anspruch 1 bis 7, **dadurch gekennzeichnet, daß** die Feinfiltration mittels Filterkerzen, Schlauch- oder Patronenfiltern erfolgt.

9. Verfahren nach Anspruch 1 bis 8, **dadurch gekennzeichnet, daß** Filterkerzen aus gesintertem Metall oder aus Keramik eingesetzt werden.

10. Vorrichtung zur Durchführung des Verfahrens gemäß Anspruch 1 bis 9, umfassend einen mit einem Wirbelbett ausgerüsteten Reaktor (2), welcher für die Einleitung der Eduktströme mit zwei Leitungen (1, 3) und für die Ableitung der Produkte mit mindestens einer Leitung (5) ausgestattet ist, **dadurch gekennzeichnet, daß** die Leitungen (1) zur Zuführung der Edukte innerhalb des Reaktors aus porösem Material gefertigt sind.

11. Vorrichtung gemäß Anspruch 10, **dadurch gekennzeichnet, daß** vor der Ableitung der Produkte über eine Leitung (5) diese mit einer Filtervorrichtung (4) aus porösem Material gefiltert werden.

12. Vorrichtung gemäß Anspruch 10 bis 11, **dadurch gekennzeichnet, daß** für die Einleitung des Ethylens und des Kreisgasstroms der Reaktor (2) mit einem Boden (12), der aus porösem gasdurchlässigem Material gefertigt, oder mit Formkörper aus porösem gasdurchlässigem Material versehen ist, ausgerüstet ist.

**Claims**

1. Method for the preparation of 1,2-dichloroethane by reacting ethene with hydrogen chloride and oxygen or an oxygen-containing gas on a copper-containing catalyst in a fluidised bed, **characterised in that** at least one of the starting material flows of hydrogen chloride and oxygen is introduced directly into the fluidised bed by way of feed pipes made from porous gas-permeable moulding, and the ethylene and circulating gas flow are introduced by way of a base that is manufactured from porous gas-permeable material or that is provided with moulding made from porous gas-permeable material.

2. Method according to claim 1, **characterised in that** the porous mouldings are made from glass or ceramics.

3. Method according to claim 1, **characterised in that** the porous mouldings are made from sintered metals.

4. Method according to claims 1 to 3, **characterised in that** the porous mouldings have pores of a diameter of from 0.5 to 50 µm.

5. Method according to claims 1 to 4, **characterised in that** in the reactor the supply of gas through the porous mouldings can take place in all directions.

6. Method according to claims 1 to 5, **characterised in that** the catalyst carried over from the fluidised bed is largely held back in the reactor by means of fine filters made from porous gas-permeable moulding.

7. Method according to claims 1 to 6, **characterised in that** particles of > 1 µm are held back by the fine filtration.

8. Method according to claims 1 to 7, **characterised in that** the fine filtration is effected by means of filter candles, tube filters or cartridge filters.

9. Method according to claims 1 to 8, **characterised in that** filter candles made from sintered metal or from ceramics are used.

10. Device for carrying out the method according to claims 1 to 9, comprising a reactor (2) that is equipped with a fluidised bed, which reactor is provided with two pipes (1, 3) for the introduction of the starting material flows and with at least one pipe (5) for drawing off the products, **characterised in that** the pipes (1) for feeding in the starting materials inside the reactor are manufactured from porous material.

11. Device according to claim 10, **characterised in that** before the products are drawn off by way of a pipe (5) they are filtered by a filter device (4) made from porous material.

12. Device according to claims 10 and 11, **character-**

**ised in that**, for the introduction of the ethylene and the circulating gas flow, the reactor (2) is provided with a base (12) that is manufactured from porous gas-permeable material or that is provided with moulding from porous gas-permeable material.

## Revendications

1. Procédé de préparation du 1,2-dichloroéthane par réaction de l'éthène avec le chlorure d'hydrogène et l'oxygène ou un gaz contenant de l'oxygène sur un catalyseur contenant du cuivre dans le lit fluidisé, **caractérisé en ce que** l'admission d'au moins un des courants d'éduit, chlorure d'hydrogène et oxygène, se réalise directement dans le lit fluidisé, par des conduites en corps moulé poreux perméable aux gaz et que l'admission de l'éthylène et du courant gazeux circulant se réalise par l'intermédiaire d'un fond qui est fabriqué en matériau poreux perméable aux gaz ou qui est pourvu d'un corps moulé en matériau poreux perméable aux gaz.

2. Procédé selon la revendication 1, **caractérisé en ce que** les corps moulés poreux sont constitués de verre ou de céramique.

3. Procédé selon la revendication 1, **caractérisé en ce que** les corps moulés poreux sont constitués de métaux frittés.

4. Procédé selon les revendications 1 à 3, **caractérisé en ce que** les corps moulés poreux possèdent des pores ayant un diamètre de 0,5 à 50 μm.

5. Procédé selon les revendications 1 à 4, **caractérisé en ce que**, dans le réacteur, l'admission des gaz peut se réaliser dans toutes les directions à travers les corps moulés poreux.

6. Procédé selon les revendications 1 à 5, **caractérisé en ce que** le catalyseur entraîné hors du lit fluidisé est retenu en grande partie par un filtre fin en corps moulé poreux dans le réacteur.

7. Procédé selon les revendications 1 à 6, **caractérisé en ce que** les particules > 1 μm sont retenues par la filtration fine.

8. Procédé selon les revendications 1 à 7, **caractérisé en ce que** la filtration fine se réalise au moyen de filtres à bougie, filtres à manche, filtres à cartouche.

9. Procédé selon les revendications 1 à 8, **caractérisé en ce que** des filtres à bougie en métal fritté ou en céramique sont mis en oeuvre.

10. Dispositif pour réaliser le procédé selon les revendications 1 à 9, comprenant un réacteur (2) équipé d'un lit fluidisé, qui est muni de deux conduites (1,3) pour l'admission des courants d'éduit et d'au moins une conduite (5) pour l'évacuation des produits, **caractérisé en ce que** les conduites (1) pour introduire les éduits à l'intérieur du réacteur sont fabriquées en matériau poreux.

11. Dispositif selon la revendication 10, **caractérisé en ce que**, avant l'évacuation des produits par une conduite (5), ceux-ci sont filtrés par un dispositif à filtre (4) en matériau poreux.

12. Dispositif selon les revendications 10 à 11, **caractérisé en ce que**, pour l'admission de l'éthylène et du courant gazeux circulant, le réacteur (2) est équipé d'un fond (12) qui est fabriqué en matériau poreux perméable aux gaz ou qui est pourvu d'un corps moulé en matériau poreux perméable aux gaz.

Fig. 1